# EUROPÄISCHE PATENTSCHRIFT

(11) **EP 4 057 917 B1**
(45) Veröffentlichungstag und Bekanntmachung des Hinweises auf die Patenterteilung: **07.02.2024**
(21) Anmeldenummer: 20807705.7
(22) Anmeldetag: 13.11.2020
(51) Int. Cl.: A61B 17/128, A61B 17/122, A61B 90/92

(54) **IMPLANTATSYSTEM**
IMPLANT SYSTEM
SYSTÈME D'IMPLANT

(30) Priorität: 15.11.2019 DE 102019130938
(43) Veröffentlichungstag der Anmeldung: 21.09.2022
(73) Patentinhaber: Aesculap AG, 78532 Tuttlingen (DE)
(72) Erfinder: PLEIL, Thomas, 78073 Bad Dürrheim (DE); SCHOLTEN, Thomas, 78532 Tuttlingen (DE)
(74) Vertreter: Hoeger, Stellrecht & Partner Patentanwälte mbB
(86) Internationale Anmeldenummer: PCT/EP2020/081993
(87) Internationale Veröffentlichungsnummer: WO 2021/094503

(56) Entgegenhaltungen:
- EP-A1- 2 457 530
- DE-A1- 19 534 831
- DE-A1- 19 837 929

## Beschreibung

Die vorliegende Erfindung betrifft ein Implantatsystem mit mindestens zwei sich unterscheidenden, farblich kodierten Implantaten und mit mindestens zwei sich unterscheidenden, farblich kodierten medizinischen Instrumenten, wobei eine farbliche Kodierung der mindestens zwei Instrumente zu einer farblichen Kodierung der mindestens zwei Implantate korrespondiert, wobei die Kodierung der mindestens zwei medizinischen Instrumente mindestens ein Kodierelement umfasst, wobei die mindestens zwei medizinischen Instrumente jeweils ein erstes Werkzeugelement und mindestens ein zweites Werkzeugelement umfassen zum gemeinsamen Handhaben eines der mindestens zwei Implantate, wobei das erste Werkzeugelement und das mindestens eine zweite Werkzeugelement relativ zueinander bewegbar und an einem distalen Ende des jeweiligen Instruments angeordnet oder ausgebildet sind, wobei die mindestens zwei Instrumente jeweils eine Betätigungseinrichtung umfassen, die an einem proximalen Ende des Instruments und mit den mindestens zwei Werkzeugelementen zusammenwirkend angeordnet oder ausgebildet ist zum Bewegen des ersten Werkzeugelements und des mindestens einen zweiten Werkzeugelements relativ zueinander infolge einer Betätigung der Betätigungseinrichtung.

Implantatsysteme der eingangs beschriebenen Art kommen insbesondere dann zum Einsatz, wenn einem Anwender die Zuordnung zwischen einem Implantat und dem dafür vorgesehenen Instrument, beispielsweise einem Applikationsinstrument vereinfacht werden soll. Hierfür dienen farbliche Kennzeichnungen, auch als Kodierungen bezeichnet.

Es ist bekannt, bei aus Titan hergestellten Instrumenten eine farbliche Kodierung mittels anodischer Oxidation zu realisieren. Eine Färbung der Instrumente auf diese Weise verblasst jedoch mit zunehmender Anzahl durchlaufener Aufbereitungszyklen oder kann sich im ungünstigsten Fall sogar farblich ändern.

Alternativ hierzu und insbesondere bei Instrumenten aus chirurgischem Stahl kommen Kodierelemente als Farbmarkierungen aus Kunststoff zum Einsatz, die an den Instrumenten angeschraubt oder mittels Presspassung befestigt sind. Allerdings bergen derartige Kodierelemente die Gefahr, dass sie sich lockern oder ganz vom Instrument lösen und dadurch verloren gehen können. Insbesondere ein Lösen des Kodierelements vom Instrument während eines operativen Eingriffs ist absolut inakzeptabel.

Eine Sicherung der Kodierelemente am Instrument mit Klebstoffen ist zwar grundsätzlich möglich, doch treten hier regelmäßig Biokompatibilitätsprobleme auf. Abgesehen hiervon ist zu berücksichtigen, dass Klebstoffe altern und dadurch ihre Funktion verlieren, wodurch die Gefahr des unerwünschten Lösens des Kodierelements vom Instrument wieder steigt.

DE19534831 A1 offenbart eine Schneidezange zum Schneiden von Implantatplatten mit einer eine Haltevorrichtung zum Halten der Implantatplatte sowie eine Halteschneidekante aufweisenden Haltezangenbacke und einer eine Stanzschneidekante aufweisenden Stanzzangenbacke, die beide über ein Backengelenk verbunden und mittels Zangengriffe betätigbar sind. D1 offenbart auch Haltestiften, die seitlich an einer Haltezangenbacke Farbkodierungen zugeordnet sind. Die Farbkodierungen gestatten eine einfache Zuordnung der Implantatplatten zu dem zugehörigen Haltestift.

Es ist daher eine Aufgabe der vorliegenden Erfindung, bei einem Implantatsystem der eingangs beschriebenen Art dessen farbliche Kodierung zu verbessern.

Diese Aufgabe wird bei einem Implantatsystem der eingangs beschriebenen Art erfindungsgemäß dadurch gelöst, dass das mindestens eine Kodierelement am ersten Werkzeugelement angeordnet ist und dass das mindestens eine zweite Werkzeugelement das mindestens eine Kodierelement in einer Arbeitsstellung des jeweiligen Instruments, in welcher das erste Werkzeugelement und das mindestens eine zweite Werkzeugelement zwischen einer ersten Extremstellung und einer zweiten Extremstellung relativ zueinander bewegbar sind, am ersten Werkzeugelement gegen eine Bewegung in Richtung auf das zweite Werkzeugelement hin sichert.

Durch die vorgeschlagene Weiterbildung des Implantatsystems werden die Werkzeugelemente insbesondere dazu genutzt, zum einen das mindestens eine Kodierelement zu halten und zum anderen das mindestens eine Kodierelement am ersten Werkzeugelement in der Arbeitsstellung zu sichern. So kann ein Verlust des mindestens einen Kodierelements, insbesondere bei einem operativen Eingriff, verhindert werden, da das mindestens eine Kodierelement, selbst wenn sich eine Verbindung zum ersten Werkzeugelement lösen würde, nicht vom ersten Werkzeugelement abfallen kann. Dies verhindert das mindestens eine zweite Werkzeugelement. Dieses bildet praktisch einen Anschlag oder eine Bewegungsbegrenzung für das mindestens eine Kodierelement in eine Richtung vom ersten Werkzeugelement weg in Richtung auf das zweite Werkzeugelement hin. Durch die vorgeschlagene Weiterbildung ist es insbesondere möglich, auf den Einsatz von Klebstoffen zu verzichten. Ein Verlust oder ein unerwünschtes Lösen des mindestens einen Kodierelements vom ersten Werkzeugelement und ein damit einhergehender Verlust, insbesondere bei einem operativen Eingriff, können so insbesondere wirksam verhindert werden. Ferner ist eine leichte Zuordnung von Implantat und zugehörigem Instrument durch die farbliche Kodierung möglich. Vorzugsweise stimmen die farblichen Kodierungen am Implantat und am zugehörigen oder korrespondierenden Instrument überein, sodass eine eindeutige Zuordnung für einen Anwender möglich ist, also welches Instrument er zur Handhabung welches Implantats einsetzen soll.

Günstig ist es, wenn jedes Instrument mindestens zwei Kodierelemente umfasst und wenn am ersten und/oder am mindestens einen zweiten Werkzeugelement mindestens eines der mindestens zwei Kodierelemente angeordnet ist. So kann insbesondere eine Unterscheidung der verschiedenen Instrumente verbessert werden. Beispielsweise kann an jedem Werkzeugelement ein Kodierelement angeordnet sein, sodass die Kodierung des Instruments stets gut sichtbar ist, und zwar insbesondere unabhängig davon, wie das Instrument abgelegt ist oder gehalten wird.

Vorteilhaft ist es, wenn das erste Werkzeugelement und das mindestens eine zweite Werkzeugelement relativ zueinander verschiebbar oder um eine Schwenkachse verschwenkbar angeordnet oder ausgebildet sind. So können insbesondere zangen- oder scherenförmige Instrumente ausgebildet werden. Beispielsweise können die relativ zueinander verschwenkbaren Werkzeugelemente auch an distalen Enden von Rohr- oder Schiebeschäften angeordnet werden. Beispielsweise kann eine Sicherung eines Kodierelements auch durch ein relativ zum Kodierelement verschiebbares zweites Werkzeugelement erreicht werden. Zum Beispiel kann dies durch eine Verschiebung des zweiten Werkzeugelements relativ zum ersten Werkzeugelement parallel zu einer Längsachse des Instruments ermöglicht werden.

Auf einfache Weise lässt sich das Instrument ausbilden, wenn das erste Werkzeugelement und das mindestens eine zweite Werkzeugelement mit einem die Schwenkachse definierenden Schwenkglied miteinander gekoppelt oder verbunden sind. So lassen sich durch die zwei oder mehr Werkzeugelemente ein Instrumentenmaul definierende Maulteile realisieren, und zwar sowohl bei einem scherenförmigen Instrument mit starren Branchen als auch bei einem Rohrschaftinstrument oder einem Schiebeschaftinstrument.

Eine Kopplung der Werkzeugelemente aneinander ist auf einfache Weise insbesondere dadurch möglich, dass das Schwenkglied in Form einer Schlussschraube oder in Form eines Niets ausgebildet ist.

Gemäß einer weiteren bevorzugten Ausführungsform der Erfindung kann vorgesehen sein, dass das erste Werkzeugelement und das mindestens eine zweite Werkzeugelement in einem Lagerungsbereich beweglich aneinander gelagert sind und dass das mindestens eine Kodierelement distalseitig des Lagerungsbereichs am ersten Werkzeugelement angeordnet ist. Beispielsweise kann der Lagerungsbereich ein Schlussbereich des Instruments sein, in dem die Werkzeugelemente beispielsweise mit einem Schwenkglied aneinander gekoppelt sind. Durch die Anordnung des mindestens einen Kodierelements distalseitig des Lagerungsbereichs ist die Kodierung des Instruments gut sichtbar. Ferner ist die Kodierung des Instruments nahe dem mit dem Instrument gehandhabten Implantat. Dies ermöglicht es einem Anwender, einfach und sicher visuell zu prüfen, ob für das jeweilige Implantat auch das passende Instrument gewählt wurde. Dies ist der Fall, wenn die farblichen Kodierungen übereinstimmen oder zueinander korrespondieren, beispielsweise dieselbe Farbe aufweisen. Eine korrekte Zuordnung ist eben dann nicht der Fall, wenn sich die farblichen Kodierungen unterscheiden, beispielsweise weil die Farbe des Kodierelements eine andere Farbe ist als die Farbe des Implantats.

Günstig ist es, wenn das erste Werkzeugelement eine Kodierelementaufnahme aufweist und wenn das mindestens eine Kodierelement in der Kodierelementaufnahme mindestens teilweise, insbesondere vollständig, kraft- und/oder form- und/oder stoffschlüssig gehalten ist. Das mindestens eine Kodierelement kann vollständig in der Kodierelementaufnahme aufgenommen sein oder teilweise aus dieser hervorstehen. Die Kodierelementaufnahme ermöglicht es insbesondere, das mindestens eine Kodierelement, insbesondere in der Arbeitsstellung, sicher am ersten Werkzeugelement zu halten. Insbesondere kann die Kodierelementaufnahme ausgebildet sein, um eine Bewegung des mindestens einen Kodierelements in einer Richtung vom mindestens einen zweiten Werkzeugelement weg zu verhindern. Dies kann durch Kraft- oder Formschluss zwischen dem mindestens einen Kodierelement und dem ersten Werkzeugelement erreicht werden. Optional zusätzlich oder alternativ kann das mindestens eine Kodierelement auch stoffschlüssig mit dem Instrument verbunden werden. So kann insbesondere eine zusätzliche Sicherung des Kodierelements am Instrument realisiert werden.

Vorteilhaft ist es, wenn das mindestens eine Kodierelement in der Kodierelementaufnahme ausschließlich kraft- und/oder formschlüssig, insbesondere klebstofffrei, oder ausschließlich stoffschlüssig gehalten ist. So kann es beispielsweise entweder komplett ohne Klebstoff oder dergleichen befestigt werden, wodurch sich eine Standzeit des Instruments verlängern lässt. Oder das Kodierelement kann ausschließlich stoffschlüssig, beispielsweise durch Kleben, Löten oder Schweißen fixiert werden, was einen Herstellungsaufwand minimiert.

Auf einfache Weise lässt sich das mindestens eine Kodierelement mit dem ersten Werkzeugelement verbinden, wenn das mindestens eine Kodierelement in die Kodierelementaufnahme eingeschraubt ist. Alternativ kann es auch formschlüssig eingepresst sein.

Vorzugsweise umfasst die Kodierelementaufnahme eine Durchbrechung. Eine Durchbrechung ermöglicht es insbesondere, dass das mindestens eine in der Kodierelementaufnahme aufgenommene und gehaltene Kodierelement gut erkennbar ist. Ein Anwender kann so direkt erkennen, welche Farbe das in der Kodierelementaufnahme gehaltene Kodierelement aufweist, wenn er auf eine äußerliche Oberfläche des ersten Werkzeugelements schaut, die vom zweiten Werkzeugelement weg weist.

Auf einfache Weise lässt sich das mindestens eine Kodierelement in der Kodierelementaufnahme festlegen, wenn die Durchbrechung ein Innengewinde aufweist. So kann das Kodierelement in die Kodierelementaufnahme eingeschraubt werden.

Vorteilhaft ist es, wenn das mindestens eine Kodierelement ein zum Innengewinde korrespondierendes Außengewinde aufweist. Eine solche Ausgestaltung erleichtert das Verbinden des mindestens einen Kodierelements mit dem ersten Werkzeugelement.

Um die Gefahr eines Lösens des mindestens einen Kodierelements vom ersten Werkzeugelement weiter zu verringern, ist es vorteilhaft, wenn sich die Durchbrechung in einer Richtung vom mindestens einen zweiten Werkzeugelement weg weisend mindestens abschnittsweise verjüngt. Sie kann sich auch auf ihrer gesamten Länge verjüngen. Die Durchbrechung bildet so insgesamt einen Anschlag und dadurch eine Sicherung für das mindestens eine Kodierelement am ersten Werkzeugelement. Eine Bewegung des ersten Werkzeugelements vom zweiten Werkzeugelement weg durch die Durchbrechung hindurch kann so sicher verhindert werden.

Die Herstellung des Instruments kann insbesondere dadurch vereinfacht werden, dass die Kodierelementaufnahme eine Kodierelementaufnahmelängsachse definiert, welche parallel oder im Wesentlichen parallel zur Schwenkachse verläuft. So kann eine Kodierung des Instruments gut erkannt werden, wenn der Lagerungsbereich des Instruments sichtbar ist.

Günstig ist es, wenn das erste Werkzeugelement eine Dicke in einer Richtung parallel zur Kodierelementaufnahme aufweist, und wenn eine Länge des Kodierelements maximal etwa der Dicke des Werkzeugelements entspricht. Insbesondere ist es so möglich, dass Kodierelement beidseitig flächenbündig, also einerseits mit einer Außenseite des ersten Werkzeugelements, die vom zweiten Werkzeugelement weg weist, und andererseits mit einer Seitenfläche des ersten Werkzeugelements, die in Richtung auf das zweite Werkzeugelement hin weist, auszubilden.

Vorteilhaft ist es, wenn die Kodierelementaufnahme eine vom mindestens einen zweiten Werkzeugelement weg weisende Sichtöffnung und eine in Richtung auf das mindestens eine zweite Werkzeugelement hin weisende Einführöffnung aufweist und wenn eine Querschnittsfläche der Sichtöffnung kleiner ist als eine Querschnittsfläche der Einführöffnung. Diese Ausgestaltung ermöglicht es insbesondere, das mindestens eine Kodierelement auf einfache Weise in der Kodierelementaufnahme gegen ein Herausfallen durch die Sichtöffnung zu sichern. Durch die Wahl der Querschnittsflächen der Sichtöffnung und der Einführöffnung kann das mindestens eine Kodierelement beispielsweise ausschließlich durch die Einführöffnung in die Kodierelementaufnahme eingebracht werden. Ein Durchtreten durch die Kodierelementaufnahme durch die Sichtöffnung hindurch ist dann praktisch nicht möglich.

Günstig ist es, wenn die Einführöffnung in der Arbeitsstellung mindestens teilweise, insbesondere vollständig, durch das mindestens eine zweite Werkzeugelement überdeckt oder verschlossen ist. Auf diese Weise kann das mindestens eine zweite Werkzeugelement das mindestens eine Kodierelement in der Kodierelementaufnahme gegen ein Herausfallen sichern, wenn das Instrument die Arbeitsstellung einnimmt.

Um das Instrument einfach und sicher reinigen zu können, ist es günstig, wenn das Instrument von der Arbeitsstellung in eine Aufbereitungsstellung, in welcher das erste Werkzeugelement und das mindestens eine zweite Werkzeugelement über eine der zwei Extremstellungen hinaus relativ zueinander bewegt sind, bringbar ist und wenn das mindestens eine zweite Werkzeugelement das mindestens eine Kodierelement, insbesondere die Einführöffnung, in der Aufbereitungsstellung freigibt. Beispielsweise können in der Aufbereitungsstellung die Werkzeugelemente relativ zueinander um 90° oder mehr verschwenkt sein. In jedem Fall sind sie in der Aufbereitungsstellung soweit derart relativ zueinander bewegt, dass das mindestens eine zweite Werkzeugelement das mindestens eine Kodierelement nicht mehr überdeckt, auch nicht teilweise. Dies kann insbesondere dadurch erreicht werden, dass das mindestens eine zweite Werkzeugelement die Einführöffnung in der Arbeitsstellung freigibt.

Vorzugsweise ist das mindestens eine Kodierelement in der Kodierelementaufnahme, insbesondere in der Aufbereitungsstellung, gesichert durch stoffschlüssiges Verbinden mit dem ersten Werkzeugelement und/oder durch plastisches Verformen zusammen mit dem ersten Werkzeugelement. Diese Weiterbildung ermöglicht es insbesondere, ein unbeabsichtigtes Lösen des mindestens einen Kodierelements in der Aufbereitungsstellung vom ersten Werkzeugelement mit großer Sicherheit zu verhindern. Stoffschlüssiges Verbinden kann insbesondere durch Schweißen oder Löten erreicht werden. Eine plastische Verformung kann insbesondere zu einem lokalen Formschluss führen, welcher eine Bewegung des mindestens einen Kodierelements relativ zur Kodierelementaufnahme verhindert.

Auf einfache Weise lässt sich das mindestens eine Kodierelement in der Kodierelementaufnahme sichern, wenn es darin durch Stauchen oder Zerstören mindestens eines Gewindegangs des Außengewindes und/oder des Innengewindes gesichert ist. Beispielsweise kann das Zerstören des Gewindegangs mittels Laserschweißen erreicht werden. Ein Stauchen mindestens eines Gewindegangs kann beispielsweise mechanisch mit einem dafür geeigneten Werkzeug erfolgen. So kann insbesondere verhindert werden, dass das mindestens eine Kodierelement aus der Kodierelementaufnahme herausgeschraubt werden kann.

Gemäß einer weiteren bevorzugten Ausführungsform der Erfindung kann vorgesehen sein, dass ein vom mindestens einen zweiten Werkzeugelement weg weisendes Ende des mindestens einen Kodierelements flächenbündig oder im Wesentlichen flächenbündig mit einer vom mindestens einen zweiten Werkzeugelement weg weisenden Seitenfläche des Werkzeugelements angeordnet oder ausgebildet ist. So kann insbesondere eine sich quer zur Kodierelementlängsachse erstreckende Stirnfläche des Kodierelements praktisch nahtlos in die Seitenfläche des ersten Werkzeugelements integriert werden. Mit anderen Worten kann so eine flächenbündige Einpassung des Kodierelements realisiert werden.

Die Montage des Implantatsystems kann insbesondere dadurch vereinfacht werden, dass das mindestens eine Kodierelement ein in Richtung auf das mindestens eine zweite Werkzeugelement hin weisend geöffnetes Werkzeugeingriffselement aufweist. Insbesondere kann das Werkzeugeingriffselement in Form eines Schlitzes, eines Innenvielkant oder eines Innenvielrund ausgebildet sein. Eine solche Ausgestaltung vereinfacht insbesondere das Einbringen, beispielsweise das Einschrauben, des mindestens einen Kodierelements in die Kodierelementaufnahme.

Vorteilhafterweise ist mindestens einen zweiten Werkzeugelement in analoger Weise wie am ersten Werkzeugelement mindestens ein Kodierelement angeordnet. Wie bereits weiter oben angegeben ist es so möglich, insbesondere beide oder alle Werkzeugelemente mit einem Kodierelement auszustatten. Ist das Kodierelement am mindestens einen zweiten Werkzeugelement angeordnet, hindert das erste Werkzeugelement das Durchtreten des Kodierelements in Richtung auf das erste Werkzeugelement hin. Zwei oder mehr Kodierelemente am Instrument anzuordnen hat insbesondere den Vorteil, dass ein Anwender unabhängig von einer Lage oder Orientierung des Instruments dessen Kodierung jederzeit gut erkennen kann.

Günstig ist es, wenn mindestens eines der mindestens zwei Instrumente mindestens zwei Kodierelemente umfasst und wenn mindestens eines der mindestens zwei Kodierelemente an der Betätigungseinrichtung angeordnet oder ausgebildet ist. Auf diese Weise kann ein Instrument insbesondere auch dann noch zuverlässig identifiziert werden, wenn das an einem der Werkzeugelemente angeordnete oder ausgebildete Kodierelement nicht mehr erkennbar ist, beispielsweise aufgrund von Verschmutzung oder wenn das Kodierelement bei einem chirurgischen Eingriff von Körpergewebe verdeckt ist. Insbesondere können an der Betätigungseinrichtung auch zwei, drei oder mehr Kodierelemente angeordnet sein, wodurch eine Zuordnung des Instruments zu einem Implantat weiter verbessert wird.

Die Handhabung des Implantatsystems kann insbesondere weiter vereinfacht werden, wenn die Betätigungseinrichtung zwei relativ zueinander bewegbare, insbesondere verschwenkbare Betätigungselemente umfasst. Eine solche Ausgestaltung der Betätigungseinrichtung ermöglicht insbesondere die Handhabung des Instruments nach Art einer Schere. Beispielsweise kann die Betätigungseinrichtung zwei relativ zueinander verschwenkbare Branchen umfassen, die starr mit den Werkzeugelementen oder über ein Kraftübertragungsglied mit diesen gekoppelt sind. So lassen sich einerseits scherenartige Instrumente ausbilden oder aber auch Rohrschaft- oder Schiebeschaftinstrumente.

Ein einfacher Aufbau der mindestens zwei Instrumente kann insbesondere dadurch erreicht werden, dass die zwei Betätigungselemente in Form starrer Branchen ausgebildet sind und dass ein distales Ende jedes Betätigungselements ein Werkzeugelement trägt oder umfasst.

Günstigerweise sind die zwei Betätigungselemente um die Schwenkachse verschwenkbar. So kann insbesondere ein einfacher Aufbau des Instruments realisiert werden.

Vorteilhaft ist es, wenn eines der mindestens zwei Kodierelemente an mindestens einem der zwei Betätigungselemente angeordnet ist. Insbesondere kann an jedem der beiden Betätigungselemente mindestens ein Kodierelement angeordnet oder ausgebildet sein. Es können an jedem Betätigungselement auch zwei oder mehr Kodierelemente angeordnet oder ausgebildet sein. So lässt sich eine Zuordnung des Instruments zu einem Implantat auch dann noch sicher vornehmen, wenn das an einem der Werkzeugelemente angeordnete oder ausgebildete Kodierelement nicht mehr erkennbar ist, beispielsweise aufgrund von Verschmutzung oder wenn das Kodierelement bei einem chirurgischen Eingriff von Körpergewebe verdeckt ist

Ferner kann es vorteilhaft sein, wenn das Instrument eine Vorspanneinrichtung umfasst zum Ausüben einer die Betätigungseinrichtung in einer Extremsteilung haltenden vorspannenden Kraft. Beispielsweise kann die Vorspanneinrichtung die zwei Betätigungselemente in einer maximal voneinander weg bewegten Extremstellung halten, in welcher die Werkzeugelemente maximal weit voneinander entfernt sind. Insbesondere kann die Extremstellung, die die Vorspanneinrichtung definiert, eine Stellung sein, bei der das mindestens eine Instrument die Arbeitsstellung einnimmt. So kann durch die Vorspanneinrichtung insbesondere sichergestellt werden, dass das mindestens eine Kodierelement am ersten Werkzeugelement gesichert ist, und zwar insbesondere dann, wenn das mindestens eine Instrument unbetätigt ist.

Vorteilhaft ist es, wenn die Vorspanneinrichtung mindestens ein Vorspannelement umfasst und wenn die zwei Betätigungselemente entgegen der Wirkung des mindestens einen Vorspannelements aufeinander zu bewegbar sind. Beispielsweise kann jedes Betätigungselement ein Vorspannelement umfassen, welches beispielsweise auch einstückig mit dem Betätigungselement ausgebildet ist, insbesondere nach Art einer Blattfeder. Freie Enden der Vorspannelemente, die dann freie Enden der jeweiligen Betätigungselemente bilden, können beispielsweise miteinander gekoppelt sein, insbesondere beweglich.

Günstig ist es, wenn die Vorspanneinrichtung deaktivierbar ist zum Überführen des mindestens einen medizinischen Instruments von der Arbeitsstellung in die Aufbereitungsstellung. Beispielsweise kann die Vorspanneinrichtung eine Extremstellung in der Arbeitsstellung definieren, in welcher die Betätigungselemente und insbesondere auch die Werkzeugelemente maximal weit voneinander entfernt sind. Beispielsweise können in der Arbeitsstellung miteinander in Eingriff stehende Vorspannelemente außer Eingriff gebracht werden, um die Betätigungselemente und die mit diesen verbundenen oder gekoppelten Werkzeugelemente von der Arbeitsstellung in die Aufbereitungsstellung zu überführen.

Gemäß einer bevorzugten Ausführungsform der Erfindung kann vorgesehen sein, dass die mindestens zwei Instrumente in Form von Clipanlegezangen ausgebildet sind. Insbesondere können sie unterschiedlich geformte Werkzeugelemente aufweisen, um so unterschiedlich große Implantate in Form von Clips handhaben zu können.

Vorteilhaft ist es, wenn die mindestens zwei Implantate in Form chirurgischer Clips ausgebildet sind. Insbesondere können sie in Form von Aneurysmenclips ausgebildet sein. Derartige Implantate können insbesondere eingesetzt werden, um Gefäße abzuklemmen.

Auf einfache und kostengünstige Weise lässt sich das Implantatsystem farblich kodieren, wenn das mindestens eine Kodierelement aus einem Kunststoff oder einem Metall ausgebildet ist.

Gemäß einer weiteren bevorzugten Ausführungsform der Erfindung kann vorgesehen sein, dass die mindestens zwei Implantate aus einem Implantatmaterial ausgebildet sind, dass die mindestens zwei korrespondierenden Kodierelemente aus einem Kodierelementmaterial ausgebildet sind und dass das Implantatmaterial und das Kodierelementmaterial identisch sind. Diese Ausgestaltung ermöglicht es insbesondere, eine farbliche Kodierung sowohl der Implantate als auch der Kodierelemente und damit der Instrumente identisch oder zumindest nahezu identisch zu realisieren. So kann eine besonders gute optische Zuordnung zwischen Instrument und Implantat ermöglicht werden.

Vorzugsweise ist eine Oberfläche der mindestens zwei Implantate und eine Oberfläche der mindestens zwei Kodierelemente mindestens teilweise, insbesondere vollständig, eingefärbt oder mit einer farbigen Beschichtung versehen. Eine derartige Ausgestaltung ermöglicht insbesondere eine sehr gute optische Zuordnung zwischen Implantaten und zugehörigen Instrumenten.

Günstig kann es sein, wenn das mindestens eine Kodierelement durchgefärbt ist oder eine farblich behandelte Oberfläche aufweist und wenn die mindestens zwei Implantate in gleicher Weise farblich kodiert sind. Werden das mindestens eine Kodierelement und die mindestens zwei Implantate in der gleichen Weise farblich gekennzeichnet, kann eine besonders gute Übereinstimmung des farblichen Eindrucks erreicht werden, was eine Zuordnung von Implantat und Instrument für den Anwender erleichtert.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Gesamtansicht eines ersten Ausführungsbeispiels eines Implantatsystems;
- Figur 2:: eine vergrößerte Teilansicht der Anordnung aus Figur 1;
- Figur 3:: eine Ansicht in Richtung des Pfeils A in Figur 2;
- Figur 4:: eine Ansicht in Richtung des Pfeils B in Figur 2;
- Figur 5:: eine Ansicht ähnlich Figur 4 eines distalen Endes eines Ausführungsbeispiels eines medizinischen Instruments in der Aufbereitungsstellung;
- Figur 6:: eine perspektivische Teilansicht eines Werkzeugelements vor dem Einsetzen eines Kodierelements;
- Figur 7:: eine Schnittansicht längs Linie 3-7 in Figur 3 bei geschlossenem Instrument; und
- Figur 8:: eine perspektivische Gesamtansicht eines weiteren Ausführungsbeispiels eines Instruments mit mindestens zwei Kodierelementen.

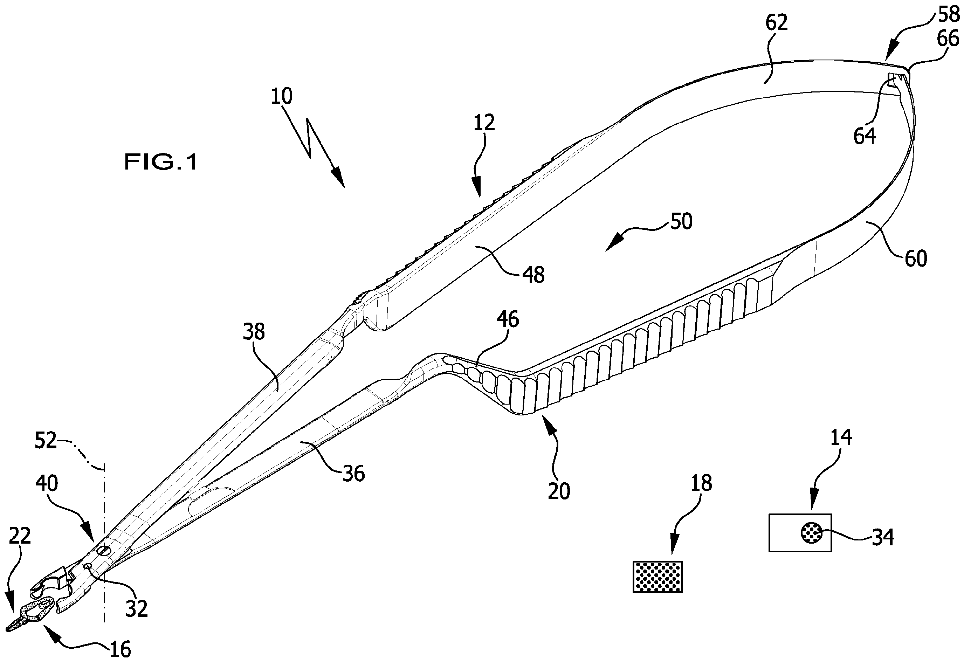

Ein Ausführungsbeispiel eines Implantatsystems ist schematisch in Figur 1 dargestellt und insgesamt mit dem Bezugszeichen 10 bezeichnet. Das Implantatsystem 10 umfasst ein erstes medizinisches Instrument 12 und ein in Figur 1 schematisch dargestelltes medizinisches Instrument 14.

Bei alternativen Ausführungsbeispielen von Implantatsystemen 10 können auch noch weitere Instrumente umfasst sein.

Das Implantatsystem 10 umfasst ferner ein erstes Implantat 16 und zweites, in Figur 1 schematisch dargestelltes Implantat 18. Das erste Instrument 12 und das zweite Instrument 14 unterscheiden sich in mindestens einem Merkmal, beispielsweise einer Größe oder Betätigungskraft.

Um die Instrumente 12 und 14 zu unterscheiden, sind sie farblich kodiert, wie nachstehend noch im Einzelnen erläutert wird.

Das Instrument 12 ist in den Figuren beispielhaft in Form einer Clipanlegezange 20 dargestellt.

Das erste Implantat 16 ist in den Figuren beispielhaft als chirurgischer Clip 22, und zwar in Form eines Aneurysmenclips 24 dargestellt.

Um die Implantate 16 und 18 voneinander unterscheiden zu können, sind sie farblich kodiert. Das erste Implantat 16 ist in einer ersten Farbe eingefärbt, das zweite Implantat 18 in einer anderen Farbe. Eine solche farbliche Kodierung der Implantate 16 und 18 und gegebenenfalls noch weiterer, vom Implantatsystem 10 umfasster Implantate, ermöglicht deren einfache Unterscheidung für einen Anwender.

Die Implantate 16 und 18 können sich insbesondere in Form und Größe sowie beispielsweise auch durch eine Schließ- oder Klemmkraft von zwei Klemmarmen 26 und 28 unterscheiden, wobei die Klemmkraft durch ein Federelement 30 des Clips 22 aufgebracht wird.

Um das jeweils passende Instrument 12, 14 zur Handhabung des jeweiligen Implantats 16, 18 sicher auswählen zu können, sind die Instrumente 12 und 14 ebenfalls farblich kodiert. Hierfür dient ein Kodierelement 32 beziehungsweise 34, welches am jeweiligen Instrument 12 beziehungsweise 14 angeordnet ist.

Das in den Figuren dargestellte Instrument 12 umfasst zwei langgestreckte Branchen 36 und 38, die in einem Lagerungsbereich 40 beweglich aneinander gelagert sind. Distale Enden der Branchen 36 und 38 bilden erste und zweite Werkzeugelemente 42 und 44, zwischen denen ein Implantat 16 oder 18 aufgenommen und gehalten werden kann.

Die Branchen 36 und 38 bilden Betätigungselemente 46 und 48 einer insgesamt mit dem Bezugszeichen 50 bezeichneten Betätigungseinrichtung des Instruments 12.

Die Betätigungselemente 46 und 48 sind relativ zueinander bewegbar, nämlich um eine Schwenkachse 52 verschwenkbar. Dadurch sind auch die Werkzeugelemente 42 und 44 um die Schwenkachse 52 verschwenkbar miteinander gekoppelt.

Die Schwenkachse 52 wird durch ein Schwenkglied 54 definiert. Dieses ist bei dem in den Figuren dargestellten Ausführungsbeispiel des Instruments 12 in Form einer Schlussschraube 56 ausgebildet. Bei einem alternativen Ausführungsbeispiel kann das Schwenkglied 54 in Form eines Niet ausgebildet sein.

Das Instrument 12 umfasst ferner eine Vorspanneinrichtung 58 zum Ausüben einer die Betätigungseinrichtung 50 in einer Extremstellung haltenden vorspannenden Kraft.

Die Vorspanneinrichtung 50 umfasst zwei Vorspannelemente 60 und 62, die derart angeordnet und ausgebildet sind, dass die zwei Betätigungselemente 46 und 48 entgegen der Wirkung der Vorspannelemente 58 und 60 aufeinander zu bewegbar sind.

Die Vorspannelemente 60 und 62 bilden proximale Endabschnitte der Betätigungselemente 46 und 48. Freie Enden 64 und 66 der Vorspannelemente 66 und 62, die damit auch freie Enden 64 und 66 der Betätigungselemente 46 und 48 bilden, stehen miteinander in Eingriff und halten das Instrument 12, wie schematisch in Figur 1 dargestellt, in einer Öffnungsstellung, die eine erste Extremstellung definiert.

Werden die Betätigungselemente 46 und 48 aufeinander zu bewegt, werden die Branchen 36 und 38 mit den an diesen ausgebildeten Werkzeugelementen 42 und 44 aufeinander zu verschwenkt, sodass sich ein Instrumentenmaul 68, welches durch die beiden Werkzeugelemente 42 und 44 definiert wird, schließt.

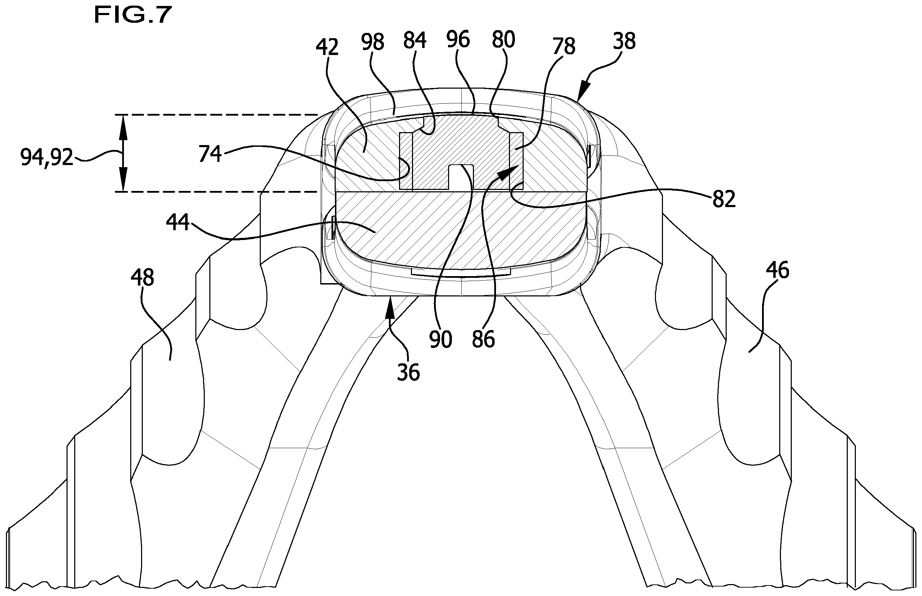

In der Arbeitsstellung des Instruments 12 stehen die freien Enden 64 und 66 der Vorspannelemente 60 und 62 in Eingriff. Eine weitere, also zweite Extremstellung in der Arbeitsstellung wird definiert durch eine maximal angenäherte Stellung der Werkzeugelemente 42 und 44, also wenn das Instrumentenmaul 68 geschlossen ist, wie dies im Schnitt in Figur 7 schematisch dargestellt ist. Eine so definierte Schließstellung definiert die zweite Extremstellung des Instrumentenmauls.

Zwischen diesen beiden definierten Extremstellungen können die Werkzeugelemente 42 und 44 relativ zueinander bewegt werden, wenn die freien Enden 64 und 66 der Vorspannelemente 60 und 62 wie beschrieben und schematisch in Figur 1 dargestellt miteinander in Eingriff stehen.

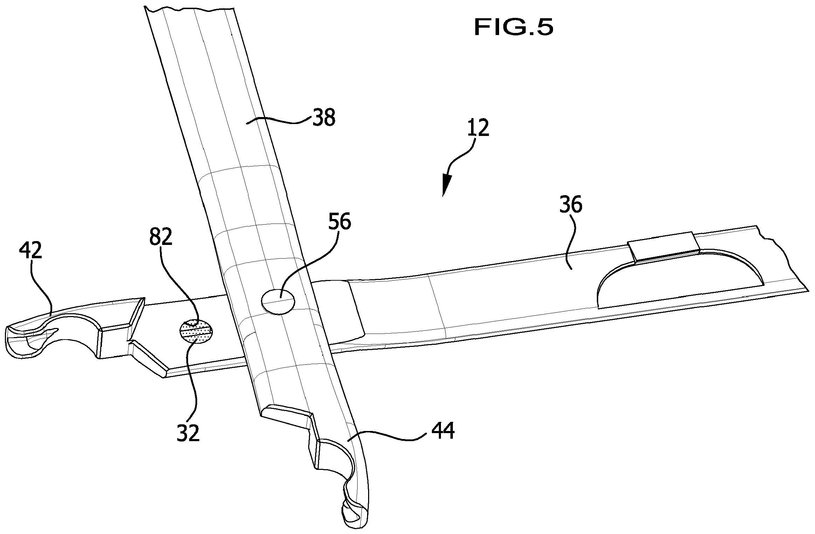

Werden die freien Enden 64 und 66 außer Eingriff gebracht, können die Branchen 36 und 38 noch weiter voneinander weg verschwenkt werden, beispielsweise in eine in Figur 5 dargestellte Position, in welcher die Werkzeugelemente 42 und 44 etwa rechtwinklig zueinander ausgerichtet sind.

Stehen die freien Enden 64 und 66 außer Eingriff, können die Branchen 36 und 38 nahezu beliebig relativ zueinander verschwenkt werden. Sind die Werkzeugelemente 42 und 44 weiter voneinander entfernt, als dies in der in Figur 1 schematisch dargestellten Öffnungsstellung, also der ersten Extremstellung, der Fall ist, nimmt das Instrument 12 eine Aufbereitungsstellung ein. In dieser Stellung wird das Instrument 12 vorzugsweise beim Waschen und Sterilisieren, also beim Aufbereiten, gelagert.

Wie beschrieben ist somit die Vorspanneinrichtung 58 deaktivierbar zum Überführen des Instruments 12 von der Arbeitsstellung in die Aufbereitungsstellung.

Das Kodierelement 32 ist am Instrument 12 in einer Kodierelementaufnahme 70 aufgenommen. Diese ist am ersten Werkzeugelement 42 distalseitig der Schwenkachse 52 und somit distalseitig des Lagerungsbereichs 40 ausgebildet.

Die Kodierelementaufnahme 70 ist in Form einer Durchbrechung 72 ausgebildet.

Die Durchbrechung 72 umfasst bei einem Ausführungsbeispiel ein Innengewinde 74.

Die Kodierelementaufnahme 70 definiert eine Kodierelementaufnahmelängsachse 76, welche parallel zur Schwenkachse 52 verläuft.

Das Kodierelement 32 weist ein zum Innengewinde 74 korrespondierendes Außengewinde 78 auf. Dies ermöglicht es, das Kodierelement 32 in die Kodierelementaufnahme 70 einzuschrauben.

Die Kodierelementaufnahme 70 weist eine vom zweiten Werkzeugelement 44 weg weisende Sichtöffnung 80 und eine in Richtung auf das zweite Werkzeugelement 44 hin weisende Einführöffnung 82 auf. Eine Querschnittsfläche der Sichtöffnung 80 ist kleiner als eine Querschnittsfläche der Einführöffnung 82. Dies wird bei den in den Figuren dargestellten Ausführungsbeispielen dadurch realisiert, dass zwischen der Einführöffnung 82 und der Sichtöffnung 80 ein Verjüngungsabschnitt 84 ausgebildet ist. Der Verjüngungsabschnitt schließt sich an einen hohlzylindrischen, mit dem Innengewinde 74 versehenen Bohrungsabschnitt 86 an.

Das Kodierelement 32 weist ferner ein in Richtung auf das zweite Werkzeugelement 44 hin weisend geöffnetes Werkzeugeingriffselement 88 in Form eines Schlitzes 90 auf.

Bei alternativen Ausführungsbeispielen ist das Werkzeugeingriffselement 88 in Form eines Innenvielkant oder eines Innenvielrund ausgebildet.

Dadurch, dass sich die Durchbrechung 72 wie beschrieben in einer Richtung vom zweiten Werkzeugelement 44 weg weisend abschnittsweise verjüngt, kann die Kodierelementaufnahme 70 das Kodierelement 32 formschlüssig aufnehmen derart, dass das Kodierelement 32 nicht durch die Sichtöffnung 80 hindurchtreten kann. Das Kodierelement 32 ist durch die Ausgestaltung der Kodierelementaufnahme 70 gegen ein Herausfallen durch die Sichtöffnung am ersten Werkzeugelement 42 gesichert.

Das Kodierelement 32 ist ferner auch gegen ein Herausfallen aus der Kodierelementaufnahme 70 durch die Einführöffnung 82 gesichert.

Ein erstes Sicherungselement bildet das zweite Werkzeugelement 44, welches die Einführöffnung 82 in der Arbeitsstellung mindestens teilweise, und in verschiedenen Stellungen auch vollständig, überdeckt beziehungsweise verschlossen hält.

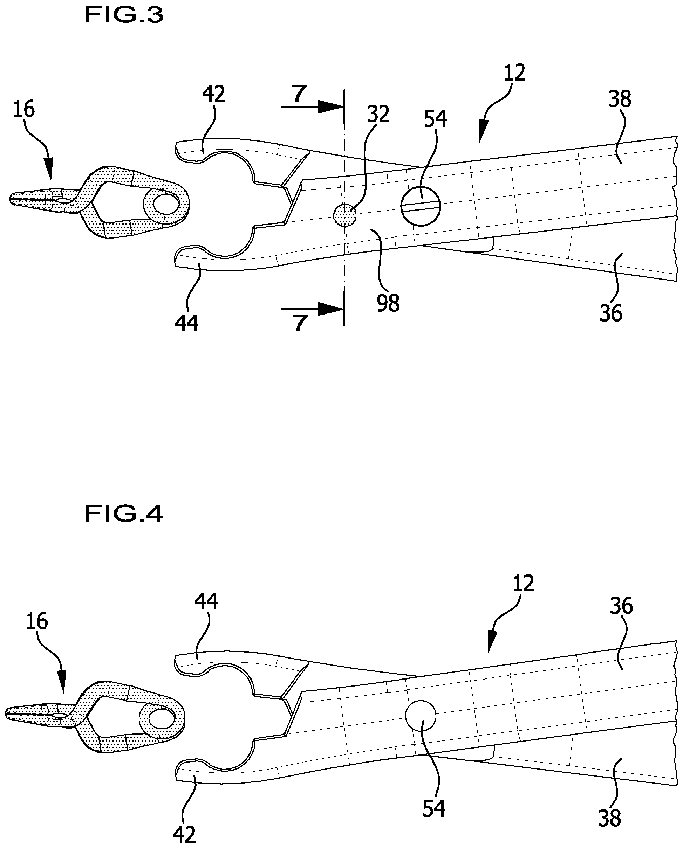

In Figur 7 ist in der Schnittansicht gut zu erkennen, dass das zweite Werkzeugelement 44 in der Schließstellung die Einführöffnung 82 vollständig überdeckt und damit verschließt. Dies ist zudem auch in Figur 4 gut zu erkennen. In der in Figur 4 dargestellten Stellung der Werkzeugelemente 42 und 44 relativ zueinander, also in der zweiten Extremstellung, in der Arbeitsstellung, also in der Stellung, in der die Vorspannelemente 60 und 62 mit ihren freien Enden 64 und 66 miteinander in Eingriff stehen, überdeckt das zweite Werkzeugelement 42 die Einführöffnung 82 vollständig, sodass das Kodierelement 32 nicht sichtbar ist.

Das zweite Werkzeugelement 44 gibt die Einführöffnung 82 jedoch in der Aufbereitungsstellung vollständig frei, wie das schematisch in Figur 5 dargestellt ist. In der Aufbereitungsstellung sind die Werkzeugelemente 42 und 44 über eine der zwei definierten Extremstellungen hinaus relativ zueinander bewegt. Bei dem in den Figuren dargestellten Ausführungsbeispiel wird wie bereits erläutert die erste Extremstellung durch eine maximale Öffnung des Instrumentenmauls 68 definiert, in welcher die freien Enden 64 und 66 der Vorspannelemente 60 und 62 in Eingriff stehen. Über diese Extremstellung hinaus sind die Werkzeugelemente 42 und 44 noch weiter voneinander weg verschwenkbar, wenn die freien Enden 64 und 66 außer Eingriff gebracht werden.

In der oben beschriebenen Weise ist das Kodierelement 32 in der Kodierelementaufnahme 70 kraft- und/oder formschlüssig gehalten, und zwar ausschließlich kraft- und/oder formschlüssig, also klebstofffrei.

Das erste Werkzeugelement 42 weist eine Dicke 92 in einer Richtung parallel zur Kodierelementaufnahmelängsachse 76 auf. Eine Länge 94 des Kodierelements 32 parallel zur Kodierelementaufnahmelängsachse 76 entspricht etwa der Dicke 92 des Werkzeugelements 42.

Damit das Kodierelement 32 in der Aufbereitungsstellung, in der das zweite Werkzeugelement 44 die Einführöffnung 82 freigibt, nicht aus der Kodierelementaufnahme 70 herausfallen kann, ist bei einem Ausführungsbeispiel das Kodierelement 32 in der Kodierelementaufnahme 70 zusätzlich gesichert. Dies ist bei einem Ausführungsbeispiel realisiert durch stoffschlüssiges Verbinden des Kodierelements 32 mit dem ersten Werkzeugelement 42 und/oder durch plastisches Verformen des Kodierelements 32 mit Werkzeugelement 42.

Diese zusätzliche Sicherung wird bei einem Ausführungsbeispiel dadurch realisiert, dass ein Gewindegang des Außengewindes 78 und/oder des Innengewindes gestaucht oder zerstört wird. Beispielsweise kann dies mittels Laserschweißen erfolgen.

Eine dauerhaft plastische Verformung des Kodierelements und/oder des Werkzeugelements 42 in dem Bereich, in dem diese miteinander im Eingriff stehen, kann eine Relativbewegung des Kodierelements 32 relativ zur Kodierelementaufnahme 70 und damit relativ zum ersten Werkzeugelement 42 dauerhaft unterbunden werden.

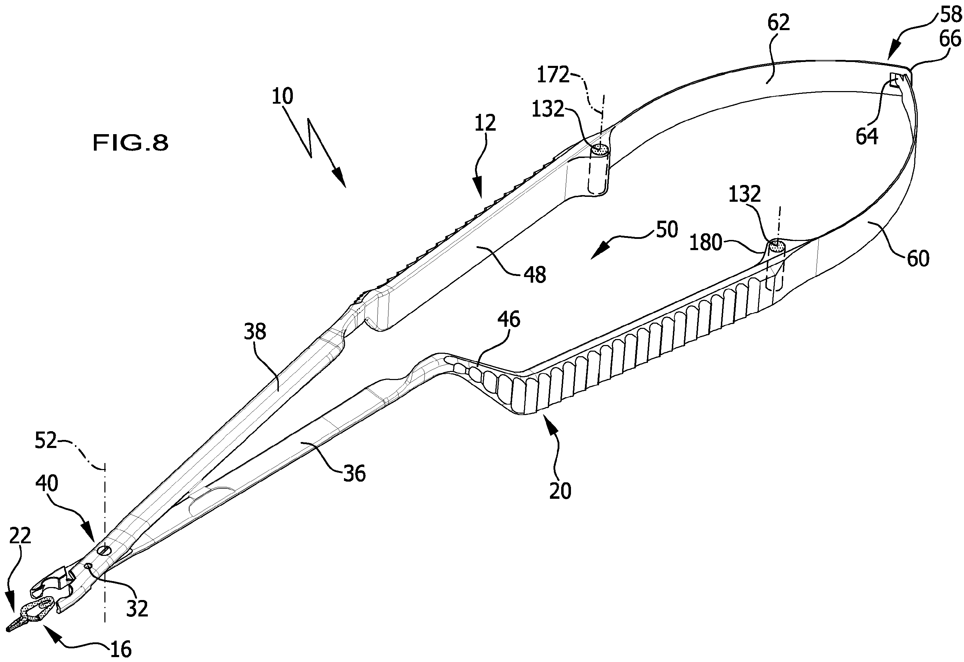

Ein weiteres Ausführungsbeispiel eines ersten medizinischen Instruments 12 eines Implantatsystems 10 ist schematisch in Figur 8 dargestellt.

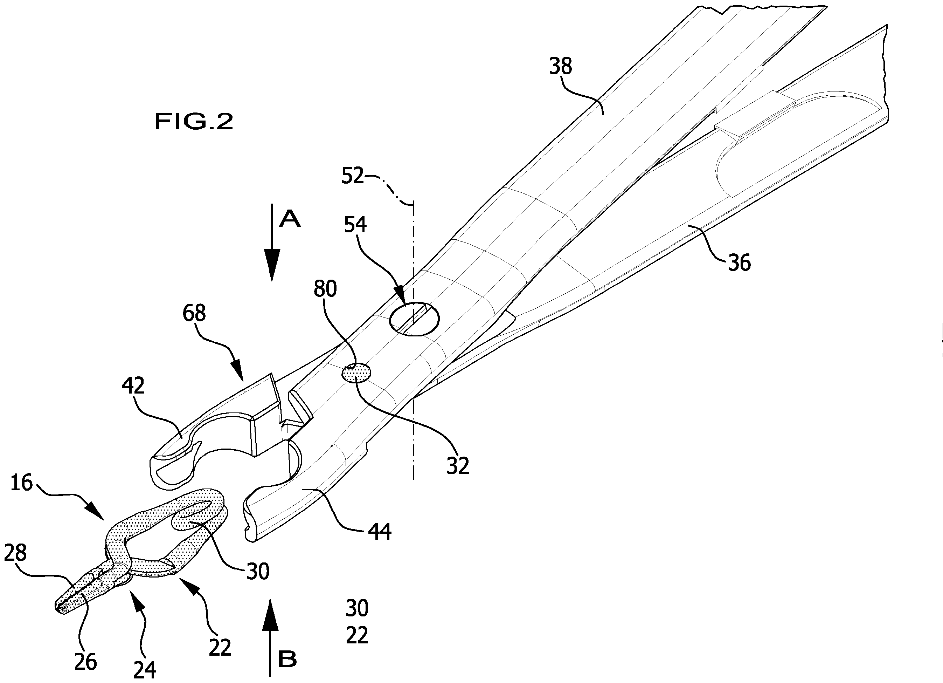

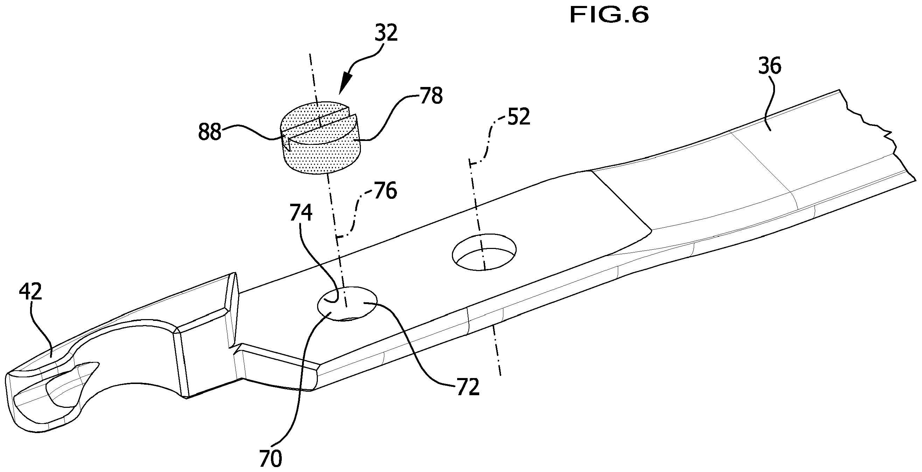

Das in Figur 8 dargestellte Ausführungsbeispiel des Instruments 12 stimmt in seinem Aufbau mit dem in Verbindung mit den Figuren 1 bis 7 erläuterten Ausführungsbeispiel des ersten medizinischen Instruments 12 im Wesentlichen überein. Daher wurden der Übersichtlichkeit wegen für identische Komponenten und Teile dieselben Bezugszeichen verwendet. Das Ausführungsbeispiel der Figur 8 unterscheidet sich jedoch vom Ausführungsbeispiel der Figuren 1 bis 7 dadurch, dass an den beiden Betätigungselementen 46 und 48 jeweils eine in Richtung auf das jeweils andere Betätigungselement 46 beziehungsweise 48 hin weisende Verdickung 180 in Form eines abgerundeten Vorsprungs ausgebildet ist. In jeder der beiden Verdickungen 180 ist eine hohlzylindrische Kodierelementaufnahme 170 ausgebildet, welche eine Längsachse 172 definiert, die parallel zur Schwenkachse 52 verläuft.

Die beiden Verdickungen 180 sind an den Betätigungselementen 46 und 48 jeweils im Übergangsbereich zu den Vorspannelementen 60 beziehungsweise 62 angeordnet beziehungsweise ausgebildet.

In die beiden Kodierelementaufnahmen 170 ist jeweils ein Kodierelement 132 eingesetzt. Die beiden Kodierelemente 132 korrespondieren zum Kodierelement 32, mithin sind sie also beispielsweise farblich identisch kodiert.

Die beiden zusätzlichen Kodierelemente 132, die an der Betätigungseinrichtung 50 angeordnet beziehungsweise ausgebildet sind, ermöglichen es einem Operateur, auch dann sicher mit dem Instrument 12 das korrespondierende erste Implantat 16 handhaben zu können, wenn das Kodierelement 32 beispielsweise verschmutzt oder anderweitig nicht einsehbar ist.

Bei einem alternativen Ausführungsbeispiel eines medizinischen Instruments 12 ist statt der zwei Verdickungen 180, wie sie in Figur 8 dargestellt sind, nur an einem der beiden Betätigungselemente 46 beziehungsweise 48 eine Verdickung 180 mit einem Kodierelement 132 angeordnet beziehungsweise ausgebildet.

Bei den in den Figuren dargestellten Ausführungsbeispielen ist ein vom zweiten Werkzeugelement 44 weg weisendes Ende 96 des Kodierelements 32 flächenbündig oder im Wesentlichen flächenbündig mit einer vom zweiten Werkzeugelement 44 weg weisenden Seitenfläche 98 des ersten Werkzeugelements 42 angeordnet oder ausgebildet.

Die Kodierelemente 32 und/oder 132 sind bei einem Ausführungsbeispiel aus einem Kunststoff ausgebildet.

Bei einem Ausführungsbeispiel sind die Kodierelemente 32 und/oder 132 aus einem Metall ausgebildet.

Die Implantate 16 und 18 sind aus einem Implantatmaterial ausgebildet. Die Kodierelemente 32, 132 und 34 sind aus einem Kodierelementmaterial ausgebildet. Bei einem Ausführungsbeispiel sind das Implantatmaterial und das Kodierelementmaterial identisch. Dies ermöglicht es insbesondere, sowohl die Kodierelement 32 und 132 beziehungsweise 34 als auch die Implantate 16 und 18 in identischer Weise farblich zu gestalten.

Oberflächen der Implantate 16 und 18 sowie Oberflächen der Kodierelemente 32, 132 und 34 sind bei einem Ausführungsbeispiel mindestens teilweise eingefärbt oder mit einer farbigen Beschichtung versehen. Bei einem Ausführungsbeispiel sind die Oberflächen der Implantate 16 und 18 sowie der Kodierelemente 32, 132 und 34 vollständig eingefärbt oder mit einer farbigen Beschichtung versehen.

Bei einem Ausführungsbeispiel sind die Kodierelemente 32, 132 und 34 durchgefärbt oder weist eine farblich behandelte Oberfläche auf. Die Implantate 16 und 18 sind in gleicher Weise farblich kodiert. So kann eine hohe Übereinstimmung der Farbkodierungen sowohl der Implantate 16, 18 als auch der Instrumente 12, 14 erreicht werden.

Die Kodierung der Instrumente 12 und 14 kann weiter verbessert werden, wenn zusätzlich zum Kodierelement 32 am ersten Werkzeugelement 42 ein weiteres Kodierelement am zweiten Werkzeugelement 44 in analoger Weise angeordnet wird. Ein am zweiten Werkzeugelement 44 angeordnetes Kodierelement wird dann gegen Herausfallen in der Arbeitsstellung durch das erste Werkzeugelement 42 gesichert.

Ein zweites Kodierelement am zweiten Werkzeugelement 44 anzuordnen hat insbesondere den Vorteil, dass auch in der schematisch in Figur 4 dargestellten Ansicht die Kodierung des Instruments 12 erkennbar wäre. So kann eine sicherere Zuordnung der Instrumente 12 beziehungsweise 14 zum jeweils korrespondierenden Implantat 16 beziehungsweise 18 realisiert werden.

Das Implantatsystem 10 kann grundsätzlich eine beliebige Anzahl an Implantaten umfassen. Insbesondere können es mehr als zwei unterschiedliche Implantate sein.

Jeder Implantattyp ist bei dem Implantatsystem 10 vorzugsweise in einer anderen Farbe farblich kodiert. Das Implantatsystem 10 kann insbesondere eine beliebige Anzahl an Instrumenten umfassen, ist also nicht auf die Instrumente 12 und 14 limitiert. Bevorzugt umfasst das Implantatsystem 10 eine Anzahl unterschiedlicher Instrumente, die der Anzahl unterschiedlicher Implantate entspricht.

Die beschriebene Kodierung der Instrumente 12 und 14 ist nicht auf die in den Figuren schematisch dargestellte Clipanlegezange 20 limitiert. Die Kodierung kann auch bei beliebigen anderen Instrumenten realisiert werden, bei denen eines der Werkzeugelemente, beispielsweise das zweite Werkzeugelement, des Instruments das Kodierelement in der Arbeitsstellung am ersten Werkzeugelement gegen einen Verlust sichert.

### Bezugszeichenliste

- 10: Implantatsystem
- 12: erstes medizinisches Instrument
- 14: zweites medizinisches Instrument
- 16: erstes Implantat
- 18: zweites Implantat
- 20: Clipanlegezange
- 22: Clip
- 24: Aneurysmenclip
- 26: Klemmarm
- 28: Klemmarm
- 30: Federelement
- 32: Kodierelement
- 34: Kodierelement
- 36: Branche
- 38: Branche
- 40: Lagerungsbereich
- 42: erstes Werkzeugelement
- 44: zweites Werkzeugelement
- 46: Betätigungselement
- 48: Betätigungselement
- 50: Betätigungseinrichtung
- 52: Schwenkachse
- 54: Schwenkglied
- 56: Schlussschraube
- 58: Vorspanneinrichtung
- 60: Vorspannelement
- 62: Vorspannelement
- 64: freies Ende
- 66: freies Ende
- 68: Instrumentenmaul
- 70: Kodierelementaufnahme
- 72: Durchbrechung
- 74: Innengewinde
- 76: Kodierelementaufnahmelängsachse
- 78: Außengewinde
- 80: Sichtöffnung
- 82: Einführöffnung
- 84: Verjüngungsabschnitt
- 86: Bohrungsabschnitt
- 88: Werkzeugeingriffselement
- 90: Schlitz
- 92: Dicke
- 94: Länge
- 96: Ende
- 98: Seitenfläche
- 132: Kodierelement
- 170: Kodierelementaufnahme
- 172: Längsachse
- 180: Verdickung

## Patentansprüche

1. Implantatsystem (10) mit mindestens zwei sich unterscheidenden, farblich kodierten Implantaten (16, 18) und **gekennzeichnet durch** mindestens zwei sich unterscheidende, farblich kodierte medizinische Instrumente (12, 14), wobei eine farbliche Kodierung der mindestens zwei Instrumente (12, 14) zu einer farblichen Kodierung der mindestens zwei Implantate (16, 18) korrespondiert, wobei die Kodierung der mindestens zwei medizinischen Instrumente (12, 14) mindestens ein Kodierelement (32, 34) umfasst, wobei die mindestens zwei medizinischen Instrumente (12, 14) jeweils ein erstes Werkzeugelement (42) und mindestens ein zweites Werkzeugelement (44) umfassen zum gemeinsamen Handhaben eines der mindestens zwei Implantate (16, 18), wobei das erste Werkzeugelement (42) und das mindestens eine zweite Werkzeugelement (44) relativ zueinander bewegbar und an einem distalen Ende des jeweiligen Instruments (12, 14) angeordnet oder ausgebildet sind, wobei die mindestens zwei Instrumente (12, 14) jeweils eine Betätigungseinrichtung (50) umfassen, die an einem proximalen Ende des Instruments (12, 14) und mit den mindestens zwei Werkzeugelementen (42, 44) zusammenwirkend angeordnet oder ausgebildet ist zum Bewegen des ersten Werkzeugelements (42) und des mindestens einen zweiten Werkzeugelements (44) relativ zueinander infolge einer Betätigung der Betätigungseinrichtung (50), wobei das mindestens eine Kodierelement (32) am ersten Werkzeugelement (42) angeordnet ist und das mindestens eine zweite Werkzeugelement (44) das mindestens eine Kodierelement (32) in einer Arbeitsstellung des jeweiligen Instruments (12, 14), in welcher das erste Werkzeugelement (42) und das mindestens eine zweite Werkzeugelement (44) zwischen einer ersten Extremstellung und einer zweiten Extremstellung relativ zueinander bewegbar sind, am ersten Werkzeugelement (42) gegen eine Bewegung in Richtung auf das zweite Werkzeugelement (44) hin sichert.

2. Implantatsystem nach Anspruch 1, **dadurch gekennzeichnet, dass** jedes Instrument (12, 14) mindestens zwei Kodierelemente (32, 34) umfasst und dass am ersten und/oder am mindestens einen zweiten Werkzeugelement (42, 44) mindestens eines der mindestens zwei Kodierelemente (32, 34) angeordnet ist.

3. Implantatsystem nach einem der voranstehenden Ansprüche, **dadurch gekennzeichnet, dass** das erste Werkzeugelement (42) und das mindestens eine zweite Werkzeugelement (44)
a) relativ zueinander um eine Schwenkachse (52) verschwenkbar angeordnet oder ausgebildet sind,
wobei insbesondere das erste Werkzeugelement (42) und das mindestens eine zweite Werkzeugelement (44) mit einem die Schwenkachse (52) definierenden Schwenkglied (54) miteinander gekoppelt oder verbunden sind,
wobei weiter insbesondere das Schwenkglied (54) in Form einer Schlussschraube (56) oder in Form eines Niets ausgebildet ist,
und/oder
b) in einem Lagerungsbereich (40) beweglich aneinander gelagert sind und dass das mindestens eine Kodierelement (32, 34) distalseitig des Lagerungsbereichs (40) am ersten Werkzeugelement (42) angeordnet ist.

4. Implantatsystem nach einem der voranstehenden Ansprüche, **dadurch gekennzeichnet, dass** das erste Werkzeugelement (42) eine Kodierelementaufnahme (70) aufweist und dass das mindestens eine Kodierelement (32, 34) in der Kodierelementaufnahme (70) mindestens teilweise, insbesondere vollständig, kraft- und/oder form- und/oder stoffschlüssig gehalten ist.

5. Implantatsystem nach Anspruch 4, **dadurch gekennzeichnet, dass** das mindestens eine Kodierelement (32, 34)
a) in der Kodierelementaufnahme (70) ausschließlich kraft- und/oder formschlüssig, insbesondere klebstofffrei, oder ausschließlich stoffschlüssig gehalten ist
und/oder
b) in die Kodierelementaufnahme (70) eingeschraubt ist.

6. Implantatsystem nach Anspruch 4 oder 5, **dadurch gekennzeichnet, dass** die Kodierelementaufnahme (70)
a) eine Durchbrechung (72) umfasst,
wobei insbesondere die Durchbrechung (72)
- ein Innengewinde (74) aufweist und das mindestens eine Kodierelement (32, 34) ein zum Innengewinde (74) korrespondierendes Außengewinde (78) aufweist
und/oder
- sich in einer Richtung vom mindestens einen zweiten Werkzeugelement (44) weg weisend mindestens abschnittsweise verjüngt,
und/oder
b) eine Kodierelementaufnahmelängsachse (76) definiert, welche parallel oder im Wesentlichen parallel zur Schwenkachse (52) verläuft,
wobei insbesondere das erste Werkzeugelement (42) eine Dicke (92) in einer Richtung parallel zur Kodierelementaufnahmelängsachse (76) aufweist und dass eine Länge (94) des Kodierelements (32, 34) maximal etwa der Dicke (92) des ersten Werkzeugelements (42) entspricht.

7. Implantatsystem nach einem der Ansprüche 4 bis 6, **dadurch gekennzeichnet, dass** die Kodierelementaufnahme (70) eine vom mindestens einen zweiten Werkzeugelement (44) weg weisende Sichtöffnung (80) und eine in Richtung auf das mindestens eine zweite Werkzeugelement (44) hin weisende Einführöffnung (82) aufweist und dass eine Querschnittsfläche der Sichtöffnung (80) kleiner ist als eine Querschnittsfläche der Einführöffnung (82),
wobei insbesondere
a) die Einführöffnung (82) in der Arbeitsstellung mindestens teilweise, insbesondere vollständig, durch das mindestens eine zweite Werkzeugelement (44) überdeckt oder verschlossen ist
und/oder
b) das Instrument (12, 14) von der Arbeitsstellung in eine Aufbereitungsstellung, in welcher das erste Werkzeugelement (42) und das mindestens eine zweite Werkzeugelement (44) über eine der zwei Extremstellungen hinaus relativ zueinander bewegt sind, bringbar ist und wobei das mindestens eine zweite Werkzeugelement (44) das mindestens eine Kodierelement (32, 34), insbesondere die Einführöffnung (82), in der Aufbereitungsstellung freigibt.

8. Implantatsystem nach einem der Ansprüche 4 bis 7, **dadurch gekennzeichnet, dass** das mindestens eine Kodierelement (32, 34) in der Kodierelementaufnahme (70), insbesondere in der Aufbereitungsstellung, gesichert ist durch stoffschlüssiges Verbinden mit dem ersten Werkzeugelement (42) und/oder durch plastisches Verformen zusammen mit dem ersten Werkzeugelement (42),
wobei insbesondere das mindestens eine Kodierelement (32, 34) in der Kodierelementaufnahme (70) gesichert ist durch Stauchen oder Zerstören mindestens eines Gewindegangs des Außengewindes (78) und/oder des Innengewindes (74), insbesondere mittels Laserschweißen.

9. Implantatsystem nach einem der voranstehenden Ansprüche, **dadurch gekennzeichnet, dass** ein vom mindestens einen zweiten Werkzeugelement (44) weg weisendes Ende des mindestens einen Kodierelements (32, 34) flächenbündig oder im Wesentlichen flächenbündig mit einer vom mindestens einen zweiten Werkzeugelement (44) weg weisenden Seitenfläche (98) des ersten Werkzeugelements (42) angeordnet oder ausgebildet ist.

10. Implantatsystem nach einem der voranstehenden Ansprüche, **dadurch gekennzeichnet, dass**
a) das mindestens eine Kodierelement (32, 34) ein in Richtung auf das mindestens eine zweite Werkzeugelement (44) hin weisend geöffnetes Werkzeugeingriffselement (88) aufweist, insbesondere in Form eines Schlitzes (90), eines Innenvielkant oder eines Innenvielrund,
und/oder
b) am mindestens einen zweiten Werkzeugelement (44) in analoger Weise wie am ersten Werkzeugelement (42) mindestens ein Kodierelement (32, 34) angeordnet ist.

11. Implantatsystem nach einem der voranstehenden Ansprüche, **dadurch gekennzeichnet, dass** mindestens eines der mindestens zwei Instrumente (12, 14) mindestens zwei Kodierelemente (32, 132) umfasst und dass mindestens eines der mindestens zwei Kodierelemente (32, 132) an der Betätigungseinrichtung (50) angeordnet oder ausgebildet ist.

12. Implantatsystem nach einem der voranstehenden Ansprüche, **dadurch gekennzeichnet, dass** die Betätigungseinrichtung (50) zwei relativ zueinander bewegbare, insbesondere verschwenkbare, Betätigungselemente (46, 48) umfasst,
wobei insbesondere
a) die zwei Betätigungselemente (46, 48) in Form starrer Branchen (36, 38) ausgebildet sind und wobei ein distales Ende jedes Betätigungselements (46, 48) ein Werkzeugelement (42, 44) trägt oder umfasst
und/oder
b) die zwei Betätigungselemente (46, 48) um die Schwenkachse (52) verschwenkbar sind
und/oder
c) an mindestens einem der zwei Betätigungselemente (46, 48), insbesondere an beiden Betätigungselementen (46, 48), eines der mindestens zwei Kodierelemente (32, 132) angeordnet oder ausgebildet ist.

13. Implantatsystem nach einem der voranstehenden Ansprüche, **dadurch gekennzeichnet, dass** die mindestens zwei Instrumente (12, 14) eine Vorspanneinrichtung (58) umfassen zum Ausüben einer die Betätigungseinrichtung (50) in einer Extremstellung haltenden vorspannenden Kraft, wobei insbesondere die Vorspanneinrichtung (58)
a) mindestens ein Vorspannelement (60, 62) umfasst und wobei die zwei Betätigungselemente (46, 48) entgegen der Wirkung des mindestens einen Vorspannelements (60, 62) aufeinander zu bewegbar sind
und/oder
b) deaktivierbar ist zum Überführen des mindestens einen medizinischen Instruments (12, 14) von der Arbeitsstellung in die Aufbereitungsstellung.

14. Implantatsystem nach einem der voranstehenden Ansprüche, **dadurch gekennzeichnet, dass**
a) die mindestens zwei Instrumente (12, 14) in Form von Clipanlegezangen (20) ausgebildet sind
und/oder
b) die mindestens zwei Implantate (16, 18) in Form chirurgischer Clips (22), insbesondere in Form von Aneurysmenclips (24), ausgebildet sind
und/oder
c) das mindestens eine Kodierelement (32, 34) aus einem Kunststoff oder einem Metall ausgebildet ist
und/oder
d) die mindestens zwei Implantate (16, 18) aus einem Implantatmaterial ausgebildet sind, dass die mindestens zwei korrespondierenden Kodierelemente (32, 34) aus einem Kodierelementmaterial ausgebildet sind und dass das Implantatmaterial und das Kodierelementmaterial identisch sind.

15. Implantatsystem nach einem der voranstehenden Ansprüche, **dadurch gekennzeichnet, dass**
a) eine Oberfläche der mindestens zwei Implantate (16, 18) und eine Oberfläche der mindestens zwei Kodierelemente (32, 34) mindestens teilweise, insbesondere vollständig, eingefärbt oder mit einer farbigen Beschichtung versehen ist
und/oder
b) das mindestens eine Kodierelement (32, 34) durchgefärbt ist oder eine farblich behandelte Oberfläche aufweist und dass die mindestens zwei Implantate (16, 18) in gleicher Weise farblich kodiert sind.

## Claims

1. Implant system (10) comprising at least two differing color-coded implants (16, 18) and **characterized by** at least two differing color-coded medical instruments (12, 14), wherein a color coding of the at least two instruments (12, 14) corresponds to a color coding of the at least two implants (16, 18), wherein the coding of the at least two medical instruments (12, 14) comprises at least one coding element (32, 34), wherein the at least two medical instruments (12, 14) each comprise a first tool element (42) and at least one second tool element (44) for jointly handling one of the at least two implants (16, 18), wherein the first tool element (42) and the at least one second tool element (44) are movable relative to one another and are arranged or formed on a distal end of the respective instrument (12, 14), wherein the at least two instruments (12, 14) each comprise an actuating device (50), which is arranged or formed on a proximal end of the instrument (12, 14) and cooperating with the at least two tool elements (42, 44) for moving the first tool element (42) and the at least one second tool element (44) relative to one another as a result of an actuation of the actuating device (50), wherein the at least one coding element (32) is arranged on the first tool element (42) and the at least one second tool element (44) secures the at least one coding element (32) in a working position of the respective instrument (12, 14), in which the first tool element (42) and the at least one second tool element (44) are movable relative to one another between a first extreme position and a second extreme position, on the first tool element (42) against a movement in the direction toward the second tool element (44).

2. Implant system in accordance with Claim 1, **characterized in that** each instrument (12, 14) comprises at least two coding elements (32, 34) and **in that** at least one of the at least two coding elements (32, 34) is arranged on the first and/or on the at least one second tool element (42, 44).

3. Implant system in accordance with any one of the preceding Claims, **characterized in that** the first tool element (42) and the at least one second tool element (44)
a) are arranged or formed so as to be pivotable relative to one another about a pivot axis (52),
wherein, in particular, the first tool element (42) and the at least one second tool element (44) are coupled or connected to one another with a pivot member (54) defining the pivot axis (52), wherein, further in particular, the pivot member (54) is configured in the form of a connecting screw (56) or in the form of a rivet,
and/or
b) are movably mounted against one another in a bearing region (40) and **in that** the at least one coding element (32, 34) is arranged on the first tool element (42) on the distal side of the bearing region (40).

4. Implant system in accordance with any one of the preceding Claims, **characterized in that** the first tool element (42) has a coding element receptacle (70) and **in that** the at least one coding element (32, 34) is at least partially, in particular completely, held in the coding element receptacle (70) in a force-locking and/or positive-locking manner and/or by material bond.

5. Implant system in accordance with Claim 4, **characterized in that** the at least one coding element (32, 34)
a) is held in the coding element receptacle (70) exclusively in a force-locking and/or positive-locking manner, in particular without adhesive, or exclusively by material bond
and/or
b) is screwed into the coding element receptacle (70).

6. Implant system in accordance with Claim 4 or 5, **characterized in that** the coding element receptacle (70)
a) comprises a perforation (72),
wherein, in particular, the perforation (72)
- has an internal thread (74) and the at least one coding element (32, 34) has an external thread (78) corresponding to the internal thread (74)
and/or
- tapers at least in sections in a direction pointing away from the at least one second tool element (44),
and/or
b) defines a coding element receptacle longitudinal axis (76), which extends in parallel or substantially in parallel to the pivot axis (52), wherein, in particular, the first tool element (42) has a thickness (92) in a direction parallel to the coding element receptacle longitudinal axis (76) and **in that** a length (94) of the coding element (32, 34) corresponds to at most about the thickness (92) of the first tool element (42).

7. Implant system in accordance with any one of Claims 4 to 6, **characterized in that** the coding element receptacle (70) has a viewing opening (80) pointing away from the at least one second tool element (44) and an insertion opening (82) pointing in the direction toward the at least one second tool element (44), and **in that** a cross sectional area of the viewing opening (80) is smaller than a cross sectional area of the insertion opening (82),
wherein, in particular,
a) the insertion opening (82) in the working position is at least partially, in particular completely, covered or closed by the at least one second tool element (44)
and/or
b) the instrument (12, 14) is bringable from the working position into a processing position in which the first tool element (42) and the at least one second tool element (44) are moved relative to one another beyond one of the two extreme positions, and wherein the at least one second tool element (44) releases in the processing position the at least one coding element (32, 34), in particular unblocks the insertion opening (82).

8. Implant system in accordance with any one of Claims 4 to 7, **characterized in that** the at least one coding element (32, 34) is secured in the coding element receptacle (70), in particular in the processing position, by material bonding to the first tool element (42) and/or by plastic deformation together with the first tool element (42), wherein, in particular, the at least one coding element (32, 34) is secured in the coding element receptacle (70) by crushing or destroying at least one thread of the external thread (78) and/or the internal thread (74), in particular by means of laser welding.

9. Implant system in accordance with any one of the preceding Claims, **characterized in that** an end of the at least one coding element (32, 34) pointing away from the at least one second tool element (44) is arranged or formed flush or substantially flush with a side face (98) of the first tool element (42) facing away from the at least one second tool element (44).

10. Implant system in accordance with any one of the preceding Claims, **characterized in that**
a) the at least one coding element (32, 34) has a tool engagement element (88) pointing in the direction toward the at least one second tool element (44), in particular in the form of a slot (90), an inner polygonal socket, or an inner multilobular socket,
and/or
b) at least one coding element (32, 34) is arranged on the at least one second tool element (44) in an analogous manner as on the first tool element (42).

11. Implant system in accordance with any one of the preceding Claims, **characterized in that** at least one of the at least two instruments (12, 14) comprises at least two coding elements (32, 132) and **in that** at least one of the at least two coding elements (32, 132) is arranged or formed on the actuating device (50).

12. Implant system in accordance with any one of the preceding Claims, **characterized in that** the actuating device (50) comprises two actuating elements (46, 48), which are movable, in particular pivotable, relative to one another,
wherein, in particular,
a) the two actuating elements (46, 48) are configured in the form of rigid branches (36, 38) and wherein a distal end of each actuating element (46, 48) bears or comprises a tool element (42, 44)
and/or
b) the two actuating elements (46, 48) are pivotable about the pivot axis (52)
and/or
c) one of the at least two coding elements (32, 132) is arranged or formed on at least one of the two actuating elements (46, 48), in particular on both actuating elements (46, 48).

13. Implant system in accordance with any one of the preceding Claims, **characterized in that** the at least two instruments (12, 14) comprise a biasing device (58) for exerting a biasing force holding the actuating device (50) in an extreme position,
wherein, in particular, the biasing device (58)
a) comprises at least one biasing element (60, 62) and wherein the two actuating elements (46, 48) are movable toward one another against the action of the at least one biasing element (60, 62)
and/or
b) is deactivatable for transferring the at least one medical instrument (12, 14) from the working position into the processing position.

14. Implant system in accordance with any one of the preceding Claims, **characterized in that**
a) the at least two instruments (12, 14) are configured in the form of clip appliers (20)
and/or
b) the at least two implants (16, 18) are configured in the form of surgical clips (22), in particular in the form of aneurysm clips (24),
and/or
c) the at least one coding element (32, 34) is made of a plastic material or a metal
and/or
d) the at least two implants (16, 18) are made of an implant material, **in that** the at least two corresponding coding elements (32, 34) are made of a coding element material, and **in that** the implant material and the coding element material are identical.

15. Implant system in accordance with any one of the preceding Claims, **characterized in that**
a) a surface of the at least two implants (16, 18) and a surface of the at least two coding elements (32, 34) is at least partially, in particular completely, colored or provided with a colored coating
and/or
b) the at least one coding element (32, 34) is colored through or has a color-treated surface and **in that** the at least two implants (16, 18) are color-coded in the same manner.

## Revendications

1. Système d'implant (10) avec au moins deux implants (16, 18) codés par couleur et se différenciant, et **caractérisé par** au moins deux instruments (12, 14) médicaux codés par couleur et se différenciant, dans lequel un codage coloré des au moins deux instruments (12, 14) correspond à un codage coloré des au moins deux implants (16, 18), dans lequel le codage des au moins deux instruments (12, 14) médicaux comprend au moins un élément de codage (32, 34), dans lequel les au moins deux instruments (12, 14) médicaux comprennent respectivement un premier élément d'outil (42) et au moins un deuxième élément d'outil (44) pour une manipulation commune de l'un des au moins deux implants (16, 18), dans lequel le premier élément d'outil (42) et le au moins un deuxième élément d'outil (44) sont agencés ou conçus de façon à pouvoir être déplacés l'un par rapport à l'autre et contre une extrémité distale de l'instrument (12, 14) respectif, dans lequel les au moins deux instruments (12, 14) comprennent respectivement un équipement d'actionnement (50) qui est agencé ou conçu contre une extrémité proximale de l'instrument (12, 14) et en coopération avec les au moins deux éléments d'outil (42, 44) pour le déplacement du premier élément d'outil (42) et du au moins un deuxième élément d'outil (44) l'un par rapport à l'autre suite à un actionnement de l'équipement d'actionnement (50), dans lequel le au moins un élément de codage (32) est agencé contre le premier élément d'outil (42) et le au moins un deuxième élément d'outil (44) sécurise le au moins un élément de codage (32) contre le premier élément d'outil (42) et dans une position de travail de l'instrument (12, 14) respectif, où le premier élément d'outil (42) et le au moins un deuxième élément d'outil (44) peuvent être déplacés l'un par rapport à l'autre entre une première position extrême et une seconde position extrême, à l'encontre d'un mouvement dans la direction allant vers le deuxième élément d'outil (44).

2. Système d'implant selon la revendication 1, **caractérisé en ce que** chaque instrument (12, 14) comprend au moins deux éléments de codage (32, 34) et **en ce qu'**au moins l'un des deux éléments de codage (32, 34) est agencé contre le premier et/ou contre le au moins un deuxième élément d'outil (42, 44).

3. Système d'implant selon l'une des revendications précédentes, **caractérisé en ce que** le premier élément d'outil (42) et le au moins un deuxième élément d'outil (44)
a) sont agencés ou conçus de façon à pouvoir être pivotés l'un par rapport à l'autre autour d'un axe de pivotement (52),
dans lequel en particulier le premier élément d'outil (42) et le au moins un deuxième élément d'outil (44) sont couplés ou reliés l'un à l'autre avec un organe de pivotement (54) définissant l'axe de pivotement (52),
dans lequel en outre en particulier l'organe de pivotement (54) est conçu sous la forme d'une vis de liaison (56) ou sous la forme d'un rivet,
et/ou
b) sont montés de façon mobile l'un par rapport à l'autre dans une zone de montage (40) et **en ce que** le au moins un élément de codage (32, 34) est agencé du côté distal de la zone de montage (40) contre le premier élément d'outil (42).

4. Système d'implant selon l'une des revendications précédentes, **caractérisé en ce que** le premier élément d'outil (42) présente une réception d'élément de codage (70) et **en ce que** le au moins un élément de codage (32, 34) est maintenu au moins partiellement, en particulier entièrement, dans la réception d'élément de codage (70) par liaison de force et/ou de forme et/ou de matière.

5. Système d'implant selon la revendication 4, **caractérisé en ce que** le au moins un élément de codage (32, 34)
a) est maintenu dans la réception d'élément de codage (70) exclusivement par liaison de force et/ou de forme, en particulier sans colle, ou exclusivement par liaison de matière,
et/ou
b) est vissé dans la réception d'élément de codage (70).

6. Système d'implant selon la revendication 4 ou 5, **caractérisé en ce que** la réception d'élément de codage (70)
a) comprend un perçage (72),
dans lequel en particulier le perçage (72)
- présente un filetage intérieur (74) et le au moins un élément de codage (32, 34) présente un filetage extérieur (78) correspondant au filetage intérieur (74)
et/ou
- se rétrécit au moins par endroits dans une direction pointant en s'éloignant depuis le au moins un deuxième élément d'outil (44)
et/ou
b) définit un axe longitudinal de réception d'élément de codage (76), lequel se déroule parallèlement ou essentiellement parallèlement à l'axe de pivotement (52),
dans lequel en particulier le premier élément d'outil (42) présente une épaisseur (92) dans une direction parallèle à l'axe longitudinal de réception d'élément de codage (76) et **en ce qu'**une longueur (94) de l'élément de codage (32, 34) correspond au maximum environ à l'épaisseur (92) du premier élément d'outil (42).

7. Système d'implant selon l'une des revendications 4 à 6, **caractérisé en ce que** la réception d'élément de codage (70) présente une ouverture d'observation (80) pointant en s'éloignant depuis le au moins un deuxième élément d'outil (44) et une ouverture d'insertion (82) pointant en allant dans la direction du au moins un deuxième élément d'outil (44) et **en ce qu'**une surface transversale de l'ouverture d'observation (80) est plus petite qu'une surface transversale de l'ouverture d'insertion (82),
dans lequel en particulier
a) l'ouverture d'insertion (82) est recouverte ou obstruée au moins partiellement, en particulier entièrement, dans la position de travail par le au moins un deuxième élément d'outil (44)
et/ou
b) l'instrument (12, 14) peut être amené depuis la position de travail jusque dans une position de préparation où le premier élément d'outil (42) et le au moins un deuxième élément d'outil (44) sont déplacés l'un par rapport à l'autre au-delà de l'une des deux positions extrêmes et dans lequel le au moins un deuxième élément d'outil (44) libère dans la position de préparation le au moins un élément de codage (32, 34), en particulier l'ouverture d'insertion (82).

8. Système d'implant selon l'une des revendications 4 à 7, **caractérisé en ce que** le au moins un élément de codage (32, 34) est sécurisé dans la réception d'élément de codage (70), en particulier dans la position de préparation, par une liaison de matière avec le premier élément d'outil (42) et/ou par déformation plastique ensemble avec le premier élément d'outil (42),
dans lequel en particulier le au moins un élément de codage (32, 34) est sécurisé dans la réception d'élément de codage (70) par refoulement ou destruction d'au moins un filet du filetage extérieur (78) et/ou du filetage intérieur (74), en particulier au moyen d'un soudage au laser.

9. Système d'implant selon l'une des revendications précédentes, **caractérisé en ce qu'**une extrémité, pointant en s'éloignant du au moins un deuxième élément d'outil (44), du au moins un élément de codage (32, 34) est agencée ou conçue affleurant ou essentiellement affleurant avec une surface latérale (98), pointant en s'éloignant du au moins un deuxième élément d'outil (44), du premier élément d'outil (42).

10. Système d'implant selon l'une des revendications précédentes, **caractérisé en ce que**
a) le au moins un élément de codage (32, 34) présente un élément de prise d'outil (88) ouvert et pointant en allant dans la direction du au moins un deuxième élément d'outil (44), en particulier sous la forme d'une fente (90), d'un polyèdre intérieur ou d'un multi-lobulaire intérieur,
et/ou
b) au moins un élément de codage (32, 34) est agencé contre le au moins un deuxième élément d'outil (44) de manière analogue à l'agencement contre le premier élément d'outil (42).

11. Système d'implant selon l'une des revendications précédentes, **caractérisé en ce qu'**au moins l'un des au moins deux instruments (12, 14) comprend au moins deux éléments de codage (32, 132) et **en ce qu'**au moins l'un des deux éléments de codage (32, 132) est agencé ou conçu contre l'équipement d'actionnement (50).

12. Système d'implant selon l'une des revendications précédentes, **caractérisé en ce que** l'équipement d'actionnement (50) comprend deux éléments d'actionnement (46, 48) pouvant être déplacés, en particulier pivotés, l'un par rapport à l'autre,
dans lequel en particulier
a) les deux éléments d'actionnement (46, 48) sont conçus sous la forme de branches rigides (36, 38) et dans lequel une extrémité distale de chaque élément d'actionnement (46, 48) porte ou comprend un élément d'outil (42, 44)
et/ou
b) les deux éléments d'actionnement (46, 48) peuvent être pivotés autour de l'axe de pivotement (52),
et/ou
c) l'un des au moins deux éléments de codage (32, 132) est agencé ou conçu contre au moins l'un des deux éléments d'actionnement (46, 48), en particulier contre les deux éléments d'actionnement (46, 48).

13. Système d'implant selon l'une des revendications précédentes, **caractérisé en ce que** les au moins deux instruments (12, 14) comprennent un équipement de précontrainte (58) pour l'exercice d'une force qui précontraint et maintient l'équipement d'actionnement (50) dans une position extrême,
dans lequel en particulier l'équipement de précontrainte (58)
a) comprend au moins un élément de précontrainte (60, 62) et dans lequel les deux éléments d'actionnement (46, 48) doivent être déplacés l'un sur l'autre à l'encontre de l'action du au moins un élément de précontrainte (60, 62)
et/ou
b) peut être désactivé pour le transfert du au moins un instrument (12, 14) médical depuis la position de travail jusque dans la position de préparation.

14. Système d'implant selon l'une des revendications précédentes, **caractérisé en ce que**
a) les au moins deux instruments (12, 14) sont conçus sous la forme de tenailles d'application de pince (20)
et/ou
b) les au moins deux implants (16, 18) sont conçus sous la forme de pinces chirurgicales (22), en particulier sous la forme de pinces d'anévrisme (24),
et/ou
c) le au moins un élément de codage (32, 34) est conçu dans une matière plastique ou dans un métal
et/ou
d) les au moins deux implants (16, 18) sont conçus dans un matériau d'implant, **en ce que** les au moins deux éléments de codage (32, 34) correspondants sont conçus dans un matériau d'élément de codage et **en ce que** le matériau d'implant et le matériau d'élément de codage sont identiques.

15. Système d'implant selon l'une des revendications précédentes, **caractérisé en ce que**
a) une surface des au moins deux implants (16, 18) et une surface des au moins deux éléments de codage (32, 34) est au moins partiellement, en particulier entièrement, colorée ou dotée d'un revêtement coloré
et/ou
b) le au moins un élément de codage (32, 34) est coloré dans la masse ou présente une surface traitée avec une couleur et **en ce que** les au moins deux implants (16, 18) sont codés avec une couleur de la même manière.
